Europäisches Patentamt

European Patent Office

Office européen des brevets

(11) Veröffentlichungsnummer: **0 215 420**
**A2**

(12) **EUROPÄISCHE PATENTANMELDUNG**

(21) Anmeldenummer: 86112431.1

(22) Anmeldetag: 08.09.86

(51) Int. Cl.⁴: **C12P 7/62** ,
//C12P11/00,C12N9/20

(30) Priorität: 16.09.85 DE 3532959

(43) Veröffentlichungstag der Anmeldung:
25.03.87 Patentblatt 87/13

(84) Benannte Vertragsstaaten:
AT BE CH DE FR GB IT LI NL

(71) Anmelder: Henkel Kommanditgesellschaft auf Aktien
Postfach 1100 Henkelstrasse 67
D-4000 Düsseldorf-Holthausen(DE)

(72) Erfinder: Lazar, Günter, Dr.
Tannenstrasse 41
D-4006 Erkrath(DE)

(54) Veresterung von 2-Methylpentansäure.

(57) Beschrieben wird ein Verfahren zur Veresterung von 2-Methylpentansäure mit primären oder sekundären Alkoholen mit 3 -12 C-Atomen, bei Temperaturen von 20 bis 40°C in Gegenwart einer Lipase als Katalysator, bei dem die Ausgangsstoffe 2-Methylpentansäure und Alkohol in Gegenwart von bis zu 20 Gew.-% Wasser mit einer Lipase in Kontakt gebracht werden, die an ein festes, im Reaktionsmedium unlösliches Trägermaterial fixiert ist.

EP 0 215 420 A2

## "Veresterung von 2-Methylpentansäure"

Die Erfindung betrifft ein schonendes Verfahren zur Herstellung von Estern der 2-Methylpentansäure.

Ester der 2-Methylpentansäure sind als Riechstoffe von Bedeutung. Sie werden beispielsweise in der DE-A-32 25 293 oder in der DE-A-33 05 560 beschrieben.

Bei Riechstoffen werden an die Herstellungsmethoden hohe Anforderungen gestellt, d.h. es muß vermieden werden, daß durch Nebenreaktionen Beiprodukte entstehen, die geruchlich stören. So besteht insbesondere bei Estern aus Carbonsäuren und empfindlichen, unter den üblichen Veresterungsbedingungen isomerisierbaren Alkohol ein Bedarf nach sehr schonenden und dabei technisch leicht durchführbaren Veresterungsverfahren. Die Veresterung mit Lipasen ist im Prinzip bekannt und wird beispielsweise in der wissenschaftlichen Literatur beschrieben. Ein Hinweis findet sich so zum Beispiel bei S. Okumora et al. in Biochimica et Biophysica Acta, 575 (1979) 156 - 165. Dort wird die Herstellung einer Reihe von Ölsäureestern beschrieben, wobei die Ausbeuten jedoch für ein Verfahren zur Riechstoffsynthese unbefriedigend sind.

Gegenstand der nicht vorveröffentlichten deutschen Patentanmeldung P 35 03 944.2 ist ein Verfahren zur Veresterung von 2-Methylpentansäure mit primären oder sekundären Alkoholen mit 3 bis 12 C-Atomen, dadurch gekennzeichnet, daß die Reaktion in einer gepufferten wäßrigen Lösung bei Temperaturen von 20 bis 40°C in Gegenwart einer Lipase als Katalysator und gewünschtenfalls von nicht wassermischbaren Lösungsmitteln durchgeführt wird.

Wenngleich das Verfahren der genannten älteren Anmeldung günstige Ergebnisse liefert, so bestand doch der Wunsch, die Standzeit der Lipase zu erhöhen und auf den Einsatz von Pufferlösungen zu verzichten. Dabei sollten die anderen günstigen Parameter des Verfahrens, nämlich die schonende Arbeitsweise bei Temperaturen nicht über 40°C und pH-Werten nicht unter 5 oder über 8,5 beibehalten werden, desgleichen die günstigen Ausbeuten bei einer Veresterung mit Lipasen.

Gegenstand der vorliegenden Weiterentwicklung der älteren Anmeldung ist somit die Weiterbildung des Verfahrens zur Veresterung von 2-Methylpentansäure mit primären oder sekundären Alkoholen mit 3 bis 12 C-Atomen, bei dem die Reaktion in einer gepufferten wäßrigen Lösung bei Temperaturen von 20 -40°C in Gegenwart einer Lipase als Katalysator und gewünschtenfalls von nichtwassermischbaren Lösungsmitteln durchgeführt wird, dadurch gekennzeichnet, daß die Ausgangsstoffe 2-Metylpentansäure und Alkohol in Gegenwart von bis zu 20 Gewichtsprozent Wasser mit einer Lipase in Kontakt gebracht werden, die an ein festes, im Reaktionsmedium unlösliches Trägermaterial fixiert ist und daß gewünschtenfalls auf die Pufferlösung verzichtet werden kann.

Als Katalysatoren im erfindungsgemäßen Verfahren eignen sich eine Vielzahl von Lipasen. So können beispielsweise Lipasen aus Aspergillus niger (z.B. Stamm NRRL 337) aus Rhizopus delemar (z.B. Stamm ATCC 34612) aus Geotrichum candidum (z.B. ATCC 34614) oder aus Penicillium cyclopium (z.B. Stamm ATCC 34613) verwendet werden. Die genannten Stämme gehören dem öffentlichen Teil der jeweils genannten Stammsammlung an.

Besonders geeignet sind Lipasen aus Candida cylindracea, auch Candida rugosa genannt. Eine Anzahl geeigneter Stämme dieser Spezies werden im öffentlichen Teil von Stammsammlungen (ATCC NRRL, DSM, JFCC, CBS) angeboten. Eine bevorzugte Lipase aus Candida cylindracea wird im US-Patent 3,189,529 beschrieben und leitet sich von einem Stamm ab, der bei ATCC unter der Nummer 14 830 hinterlegt und öffentlich zugänglich ist. Für die technische Durchführung ist eine von der Firma Meito Sangyo Co. Ltd. erhältliche kommerzielle Lipase mit der Bezeichnung Lipase OF 360 besonders geeignet. Bezüglich der näheren Charakterisierung von Lipasen aus Candida cylindracea sei weiterhin auf den Artikel von N. Tomizuka et al in Agr. Biol. Chem., Vol. 30, No. 11, Seite 1090 - (1966) verwiesen.

Die Fixierung von Lipasen an Trägern ist im Prinzip bekannt. Unter Fixierung sei hier zum einen die Adsorption durch physikalische Kräfte zum andern auch die chemische Bindung über ionische oder kovalente Bindungen verstanden. Die Immobilisierung von Lipasen wird beispielsweise beschrieben von Y. Kimura et al. in Eur. J. Appl. Micorbiol. Biotechnol. (1983) 17:107 oder von R.A. Wisdom et al. in Enzyme Microb. Technol., 1984, Vol. 6, 443. Für das erfindungsgemäße Verfahren eignen sich zum einen Lipasen, die durch Adsorption an anorganische oder organische, nicht wasserlösliche Träger erhalten worden sind. Derartige immobilisierte Lipasen werden beispielsweise hergestellt, indem man wäßrige Lipaselösungen in Suspensionen des Trägermaterials einbringt und mit einem Fällungsagens, beispielsweise eisgekühltem Ethanol oder Aceton versetzt. Als Trägermaterialien kommen eine Vielzahl von Stoffen in Frage, doch hat der Fachmann im Einzelfall die Eignung zu prüfen. Geeignet sind anorganische Materialien, vorzugsweise Diatomeen-Erden und unter diesen insbesondere Produkte, die unter der Bezeichnung Celite im Handel erhältlich sind, andererseits sind organische Polymere geeignet.

Nach einer besonders bevorzugten Ausführungsform der Erfindung werden trägergebundene Lipasen eingesetzt, die wie folgt hergestellt worden sind:

Zu einer Suspension des Trägermaterials wird die Lipaselösung gegeben. Die so erhaltene lipasehaltige Suspension wird eingefroren und gefriergetrocknet.

Nach einer weiteren Ausführungsform der Erfindung können auch trägerfixierte Lipasen eingesetzt werden, die kovalent an ein anorganisches Material oder organisches Polymeres gebunden sind. Geeignete Methoden zur Fixierung sind in der eingangs erwähnten Arbeit von Y. Kimura et al. beschrieben. So können beispielsweise Lipasen in Polyurethane eingebaut werden. Auch hydrophobe fotovernetzbare Harze sind geeignete Träger. Desgleichen können auch hydrophile vernetzbare, beispielsweise fotovernetzbare Systeme eingesetzt werden oder es können Gläser oder andere anorganische Träger verwendet werden, an die über eine Zwischengruppe (Spacer) das Enzym kovalent gebunden ist.

Das erfindungsgemäße Verfahren eignet sich besonders für die Veresterung von empfindlichen Alkoholen, die bei üblichen Veresterungsbedingungen, also z.B. saurer oder basischer Katalyse zu Umlagerungsreaktionen oder zur Bildung von Nebenprodukten neigen. So können neben Estern von gesättigten Alkoholen, insbesondere Ester von ein-oder mehrfach olefinisch ungesättigten primären Alkoholen oder aromatisch ungesättigten Alkoholen hergestellt werden. Die vorzugsweise primären Alkohole können dabei linear oder verzweigt oder auch carbocyclisch sein. Derartige Alkohole sind beispielsweise Propenylalkohol, die isomeren Butenylalkohole, Hexenylalkohole, Nonenylalkohole; speziell 3-Methyl-2-butenylalkohol, 3-Methyl-3-butenylalkohol, Cyclohexenylalkohol, 3,5,5-Trimethyl-2-cyclohexenylalkohol, 2,4(5)-Dimethyl-4-cyclo hexenyl-methylalkohol, Benzylalkohol, 3-Phenyl-2-Propenylalkohol, insbesondere aber der Prenylalkohol (ungesättigter $C_5$-Alkohol) Cis-Hex-3-en-1-ol, 3-Methyl-but-3-en-1-ol, 2-Methyl-butan-1-ol oder auch der Zimtalkohol. In allen diesen Fällen werden nach dem erfindungsgemäßen Verfahren keine störenden Nebenprodukte gebildet.

Nach dem verbesserten Verfahren werden die Ausgangsstoffe ohne Pufferlösung aber in Gegenwart von Wasser mit der trägerfixierten Lipase in Berührung gebracht. Die Wassermenge ist dabei so zu wählen, daß bis zu etwa 20 Gew.-% Wasser, bezogen auf die Ausgangsstoffe, eingesetzt werden. Vorzugsweise wird jedoch nur soviel Wasser verwendet, wie sich in den Ausgangsstoffen löst - (es werden also wassergesättigte Mischungen der Ausgangsstoffe eingesetzt). So werden beispielsweise im Falle des Prenylesters etwa 10 Gew.-% Wasser eingesetzt. Das Wasser kann den Ausgangsverbindungen direkt zugegeben werden oder aber es kann in einer Vorstufe über eine mit feuchtem Celite gefüllte Säule kontinuierlich eingegeben werden. In jedem Falle ist darauf zu achten, daß zumindest 1 Gew.-%, insbesondere mindestens 5 Gew.-%, zugegegen sind.

Zur Erzielung hoher Umsätze in akzeptablen Reaktionszeiten wird eine der Ausgangskomponenten also Säure oder Alkohol im Überschuß eingesetzt. Es ist daher möglich, mit einem Säure-zu Alkoholmolverhältnis zwischen 1 : 5,5 und 1 : 0,5 zu arbeiten. Dabei ist es bevorzugt, entweder den Alkohol oder aber die Säure in einem Überschuß von 10 bis 50 mol %, bezogen auf den anderen Reaktionspartner, einzusetzen.

Das erfindungsgemäße Verfahren ergibt die Reaktionsprodukte in hohen Ausbeuten.

Die immobilisierte Lipase zeichnet sich durch extrem hohe Standfestigkeit aus. So kann die Reaktion im Falle des Prenylesters beispielsweise über einen Zeitraum von 650 Stunden geführt werden, ohne daß der Veresterungsgrad unter etwa 84 % sinkt. Damit ist es möglich, die Reaktion kontinuierlich, beispielsweise im Rohrreaktor zu führen.

Beispiele

Beispiel 1

Immobilisierung der Lipase

10 g Lipase OF (Meito Sangyo) wurden in 50 ml destilliertem Wasser gelöst. Diese Lösung wurde einer Celite (Serva 535)-Suspension in destilliertem Wasser (200 g Celite/700 ml $H_2O$) unter Rühren zugesetzt. Der Ansatz wurde bei -20°C tiefgefroren und dann gefriergetrocknet.

Das Lipase-Celite-Lyophilisat wurde direkt im Säulenreaktor zur Veresterung eingesetzt.

Beispiel 2

Veresterung

100 g immobilisierte Lipase (5 g Lipase/100 g Celite) wurden in eine 51 x 2,5 cm Säule gefüllt. Eine wassergesättigte Mischung (ca. 10 Gew.-% Wasser) aus 2-Methylpentansäure und Prenylalkohol = 1/1 (volumengleich) wurde mit unterschiedlichen Geschwindigkeiten über die Säule gepumpt und die Reaktionsprodukte im Fraktionssammler aufgefangen. Nach einer Anlaufphase von einigen Stunden betrug der Veresterungsgrad ca. 84 % bei einer Durchflußrate von 0,6 ml/h. Selbst nach 650 h wurde keine Abnahme des Veresterungsgrades gefunden.

Werden keine wassergesättigten Ausgangsreagenzien eingesetzt, kann eine Mischung aus 2-Methylpentansäure und Prenylalkonol über eine wassergesättigte Celite-Vorsäule 20 × 2,5 cm und erst dann über die Reaktionssäule gepumpt werden.

## Ansprüche

1. Verfahren zur Veresterung von 2-Methylpentansäure mit primären oder sekundären Alkoholen mit 3 -12 C-Atomen, bei Temperaturen von 20 bis 40°C in Gegenwart einer Lipase als Katalysator, dadurch gekennzeichnet, daß die Ausgangsstoffe 2-Methylpentansäure und Alkohol in Gegenwart von bis zu 20 Gew.-% Wasser mit einer Lipase in Kontakt gebracht werden, die an ein festes, im Reaktionsmedium unlösliches Trägermaterial fixiert ist.

2. Verfahren nach Anspruch 1, dadurch gekennzeichnet, daß man als Lipase eine Lipase des Mikroorganismus Candida cylindracea einsetzt, insbesondere ein unter der Bezeichnung Lipase OF 360 von Meito Sangyo Co. Ltd., Japan erhältliches Produkt.

3. Verfahren nach den Ansprüchen 1 und 2, dadurch gekennzeichnet, daß man 2-Methylpentansäure mit primären Alkoholen verestert, die linear, verzweigt, cyclisch, olefinisch ungesättigt und/oder aromatisch ungesättigt sind.

4. Verfahren nach den Ansprüchen 1 bis 3, dadurch gekennzeichnet, daß man 2-Methylpentansäure mit Prenylalkohol, cis-Hex-3-en-1-ol, 3-Methyl-but-3-en-1-ol, 2-Methyl-butan-1-ol oder Zimtalkohol verestert.

5. Verfahren nach den Ansprüchen 1 bis 4, dadurch gekennzeichnet, daß die Lipase an ein anorganisches Material, insbesondere Diatomeen-Erde oder ein organisches Polymeres fixiert ist.

6. Verfahren nach den Ansprüchen 1 bis 5, dadurch gekennzeichnet, daß man eine Lipase einsetzt, die durch Eintrag einer Lipaselösung in eine wäßrige Suspension des Trägermaterials und anschließende Gefriertrocknung fixiert worden ist.

7. Verfahren nach den Ansprüchen 1 bis 6, dadurch gekennzeichnet, daß die Reaktionspartner wassergesättigt, über die trägerfixierte Lipase geleitet werden.